# EUROPÄISCHE PATENTSCHRIFT

(11) **EP 2 329 853 B1**
(45) Veröffentlichungstag und Bekanntmachung des Hinweises auf die Patenterteilung: **22.07.2015**
(21) Anmeldenummer: 10187880.9
(22) Anmeldetag: 18.10.2010
(51) Int. Cl.: A61L 31/02, A61L 31/08, A61L 31/14, A61L 27/04, A61L 27/30

(54) **Beschichtete Eisenbasislegierung für medizinische Implantate**
Implant and method for manufacturing same
Alliage à base de fer revêtu pour implants médicaux

(30) Priorität: 10.11.2009 US 259667 P
(43) Veröffentlichungstag der Anmeldung: 08.06.2011
(73) Patentinhaber: Biotronik VI Patent AG, 6341 Baar (CH)
(72) Erfinder: Bayer, Ullrich, 18211, Admannshagen-Bargeshagen (DE)
(74) Vertreter: Lindner-Vogt, Karin L.

(56) Entgegenhaltungen:
- EP-A2- 2 087 915
- WO-A1-2010/102549
- WO-A2-2007/082147
- DATABASE WPI Week 200971 Thomson Scientific, London, GB; AN 2009-Q05718 XP002717485, & CN 101 549 170 A (LIFETECH SCI SHENZHEN CO LTD) 7. Oktober 2009 (2009-10-07)

## Beschreibung

Die vorliegende Erfindung betrifft ein Verfahren zur Herstellung eines Implantats, insbesondere einer intraluminalen Endoprothese, mit einem Implantatkörper enthaltend biodegradierbares metallisches Material, vorzugsweise Eisen, insbesondere eine Eisenlegierung, sowie ein entsprechendes Implantat.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, orthopädische Implantate wie Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heute werden als Implantate besonders häufig Stents eingesetzt, die zur Behandlung von Stenosen (Gefäßverengungen) dienen. Sie weisen einen Körper in der Form eines ggf. durchbrochenen rohrförmigen oder hohlzylinderförmigen Grundgitters auf, das an beiden Längsenden offen ist. Der Implantatkörper einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können. Ein Ansatz zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren vorwiegend auf Legierungen von Magnesium und Eisen. Die vorliegende Erfindung bezieht sich vorzugsweise auf Implantate, deren biodegradierbarer Werkstoff des Implantatkörpers zumindest teilweise ein Metall enthält, vorzugsweise Eisen und insbesondere eine Eisenbasislegierung (im Folgenden kurz: Eisenlegierung).

Bei der Realisierung biodegradierbarer Implantate wird angestrebt, die Degradierbarkeit entsprechend der angestrebten Therapie bzw. der Anwendung des jeweiligen Implantats (koronar, intracranial, renal etc.) zu steuern. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, die Durchlässigkeit des Gefäßes zu gewährleisten, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Implantate mit einer Eisenlegierung, insbesondere eisenhaltige Stents, sind besonders kostengünstig und einfach herstellbar. Beispielsweise für die Behandlung von Stenosen verlieren diese Implantate allerdings ihre mechanische Integrität bzw. Stützwirkung erst nach einem vergleichsweise langen Zeitraum, d.h. erst nach einer Verweildauer in dem behandelten Organismus von ca. zwei Jahren. Dies bedeutet, dass sich der Kollapsdruck bei eisenhaltigen Implantaten für die gewünschten Anwendungen über der Zeit zu langsam verringert.

Im Stand der Technik sind bereits unterschiedliche Mechanismen der Degradationskontrolle von Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material beruhen. Diese führen dazu, dass die Degradationszeit verlängert wird. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Andere Lösungen basieren darauf, Legierungsbestandteile des biodegradierbaren Materials des Implantatkörpers gezielt zu verändern. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist insbesondere bei Implantaten mit einem Körper enthaltend eine Eisenlegierung eine zu spät einsetzende bzw. eine zu große Variabilität der Degradation des Implantats.

EP 2 087 915 offenbart ein Implant mit einem Grundkörper, der vollständig oder teilweise aus einer biokorrodieren Eisenlegierung besteht.

WO 2007/082147 offenbart eine biodegradierbare Endoprothese und ein Verfahren zur Herstellung derselben. Die Endoprothese weist einen ersten metallischen Bereich mit einer ersten Degradationsrate und einen zweiten metallischen Bereich mit einer zweiten Degradationsrate auf, wobei die erste und die zweite Degradationsrate unterschiedlich sind.

CN 101549170 offenbart ein Verfahren zur Herstellung einer Gefäßstütze, die im menschlichen Körper absorbierbar ist, welches die folgenden Schritte einschließt:
i) Formen der Stütze
ii) Reinigen und Vorpolieren der Stütze
iii) Durchführung eines Verfahrens zur Oberflächenlegierung der Stütze und
iv) Durchführung eines Feinabschlusses nach der Oberflächenbehandlung.

WO2010102549 (veröffentlicht am 16.09.2010) beschreibt oberflächenmodifizierte Eisenstents.

Folglich besteht die Aufgabe der vorliegenden Erfindung darin, ein kostengünstiges Verfahren zur Herstellung eines Implantats anzugeben, das eine Degradation des Implantats im gewünschten Zeitfenster bewirkt, insbesondere bei Implantaten mit einer Eisenbasislegierung in einem kürzeren Zeitraum. Dabei soll die Degradation zu einem kontrollierbaren Zeitpunkt stattfinden. Entsprechend besteht die Aufgabe der Erfindung außerdem darin, ein derartiges Implantat zu schaffen.

Die obige Aufgabenstellung wird durch ein Implantat gelöst, bei dem mindestens ein Teil der Oberfläche der ein biodegradierbares metallisches Material enthaltenden Implantatkörpers eine Beschichtung aufweist, welche aus einer Zusammensetzung enthaltend Calcium gebildet ist.

Bei der vorliegenden Erfindung umfasst der Körper des Implantats mindestens einen Teil des Implantats, vorzugsweise den Hauptteil des Implantats, welcher die mechanische Integrität des Implantats bewirkt.

Die Zusammensetzung der Beschichtung kann entweder ganz oder teilweise aus Calcium bestehen oder Verbindungen enthalten, die Calcium aufweisen. Vorzugsweise ist der Anteil von Strontium in der Beschichtung etwa 10 bis 20 Gew.% und/oder der Anteil von Calcium in der Beschichtung etwa 5 bis 10 Gew.%. Diese Werte gelten für Schichtdicken von bis zu 5 µm.

Das erfindungsgemäße Implantat hat den Vorteil, dass die Degradationsgeschwindigkeit wesentlich erhöht wird. Dadurch verkürzt sich die mittlere Verweilzeit des Implantats im behandelten Organismus und die Verstoffwechselung des Materials des Implantatkörpers wird beschleunigt. Zusätzlich ist bei einem solchen erfindungsgemäßen Implantat, insbesondere bei der Anwendung als orthopädisches Implantat, die Biokompatibilität erhöht, da die bei der Degradation freigesetzten Strontiumverbindungen das Knochenwachstum günstig beeinflussen und damit die Einwachskinetik des Implantats verbessern.

Die erhöhte Degradationsgeschwindigkeit wird dadurch erreicht, dass Strontium und Calcium aufgrund ihres großen Abstands zum Eisen in der elektrochemischen Spannungsreihe als hochwirksame Lokalelemente wirken und deshalb sehr schnell korrodieren. Diese Korrosion bewirkt, dass nach der Auflösung der Beschichtung die Oberfläche des Implantatkörpers so zerklüftet ist, dass eine verstärkte Flächenkorrosion und zunehmend auch eine Spaltkorrosion auftreten. Somit sinkt die integrale Verweilzeit der erfindungsgemäßen Implantate im behandelten Organismus im Vergleich zu unbeschichteten Implantaten.

In einem bevorzugten Ausführungsbeispiel enthält der Körper des Implantats vorzugsweise ein degradierbares metallisches Material, vorzugsweise überwiegend Eisen, insbesondere mehr als 80 Gew.% Eisen, besonders bevorzugt mindestens 99 Gew.% Eisen, insbesondere in einer Legierung. Als weitere metallische Materialien können alternativ oder zusätzlich Mangan, Zink und/oder Wolfram eingesetzt werden. Diese Implantate werden, da sie kostengünstig herstellbar sind, besonders gern für die Behandlung von Erkrankungen des menschlichen oder tierischen Organismus eingesetzt. Insbesondere bei eisenhaltigen Implantaten führt die erfindungsgemäße Beschichtung zu einer verringerten Degradationsdauer. Hierdurch wird eine Lücke zwischen den degradierbaren und nicht degradierbaren Legierungen für Implantate geschlossen.

Eine hinsichtlich der Kosten und in Bezug auf die gewünschte Degradationsdauer sinnvolle Schichtdicke der Beschichtung mit einer Zusammensetzung enthaltend mindestens ein Element der Gruppe bestehend aus Strontium und Calcium (im Folgenden kurz: Sr/Ca-Beschichtung) ergibt sich bei einer Dicke von etwa 0,5 µm bis etwa 10 µm, vorzugsweise etwa 1 µm bis etwa 5 µm der Sr/Ca-Beschichtung.

Um eine lokale Variation der Degradation des Implantatkörpers sowie eine größere Eindringtiefe von Strontium und Calcium in den Implantatkörper zu erreichen, weist das Implantat in einer besonders bevorzugten Ausführungsform in der Beschichtung und in dem Implantatkörper implantierten Stickstoff auf. Die Implantation wird durch einen Beschuss des mit der Sr/Ca-Beschichtung versehenen Implantats mittels Stickstoffionen erreicht. Vorzugsweise beträgt der integrale Stickstoffanteil von der Oberfläche der Sr/Ca-Beschichtung bis in eine Tiefe von 5 µm zwischen 0,1 Gew.% und 1,5 Gew. %. Hierbei wird der integrale Stickstoffanteil berechnet, indem die Gesamtmasse des enthaltenen Stickstoffs in dem sich von der Oberfläche bis in die genannte Tiefe erstreckenden Volumen in das Verhältnis zur Masse des gesamte Stoffgemischs in dem gleichen Volumen gesetzt wird. Die Stickstoffkonzentration kann bis in eine Tiefe von 200 nm mittels XPS und bis in größere Tiefen mittels EDX nachgewiesen werden.

Durch diese nachträglich ausgeführte Implantation des Implantats mit Stickstoffionen werden Sr- und Ca-Ionen thermisch angeregt und diffundieren deshalb in das Gitter des Implantatkörper-Werkstoffs, vorzugsweise in das Eisengitter, hinein. Dadurch entsteht, abhängig von der jeweils durchgeführten Implantation und deren Parameter, ein Elementgradient, der sich über die oberflächennahen Bereiche des Implantatkörpers sowie die Sr/Ca-Beschichtung erstreckt. Bei einer sehr hohen Implantationsrate liegt ein vergleichsweise hoher Gehalt an Strontium- und/oder Calcium-Ionen im Implantatkörper vor, wobei die Sr- oder Ca-Ionen bei einer hohen Implantationsrate auch vergleichsweise tief in den Implantatkörper eindringen. Insgesamt nimmt der Gehalt an Strontium- und/oder Calcium-Ionen über die betroffenen Bereiche des Implantatkörpers nach außen und durch die Beschichtung hindurch nach außen zu. Die Änderung des Gehalts über den angegebenen Bereich ist jedoch abhängig von der Implantationsrate.

Insbesondere vorteilhaft ist außerdem, auf der Sr/Ca-Beschichtung zusätzlich eine zweite Beschichtung enthaltend ein sauer abbaubares Polymer vorzusehen, beispielsweise ein Polylactid, ein Polyglycosid oder ein Copolymer aus diesen, besonders bevorzugt PLLA oder PLGA, oder ein Blend aus den genannten Polymeren. Dieses Polymer kann zusätzlich eine pharmazeutisch aktive Substanz enthalten. Die bei der Degradation des Polymers erzeugte Verringerung des pH-Werts im Bereich der Implantatoberfläche stellt einen zusätzlichen Beschleunigungsfaktor für die Korrosion, insbesondere bei einem Implantat mit einer Eisenlegierung, dar.

Hierbei wird unter einer "pharmazeutisch aktiven Substanz" (oder therapeutisch aktive oder wirksame Substanz) im Sinne der Erfindung wird ein pflanzlicher, tierischer oder synthetischer Wirkstoff (Medikament) oder ein Hormon verstanden, das in geeigneter Dosierung als Therapeutika zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Umgebung des Implantats hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können beispielsweise aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Proteine, der Hormone, der Insuline, der Zytostatika, der Peptide, der Vasodilatatoren (wie beispielsweise Sartane) und der antiproliferativ wirkenden Stoffe, der Taxole oder Taxane, hier vorzugsweise Paclitaxel oder Sirolimus, bestehen. Ferner wird die obige Aufgabenstellung gelöst durch ein Verfahren umfassend die folgenden Schritte:
a) Bereitstellen des Implantatkörpers,
b) Beschichtung des Implantatkörpers mit einer Zusammensetzung enthaltend mindestens ein Element der Gruppe bestehend aus Strontium und Calcium, wobei die Beschichtung in Schritt b) durch Abscheidung aus der Gasphase erfolgt.

Mittels eines derartigen Verfahrens wird auf sehr kostengünstige Weise eine oben näher erläuterte Erhöhung der Degradationsgeschwindigkeit von Implantaten erreicht.

Die Beschichtung erfolgt durch Abscheidung aus der Gasphase. Hierzu werden die Elemente Strontium und/oder Calcium in einer Behandlungskammer wahlweise mit oder ohne Vakuumunterstützung thermisch verdampft. Die in der Behandlungskammer angeordneten medizinischen Implantate werden hierdurch allseitig oder, wenn eine entsprechende Abdeckung aufgebracht ist, teilweise mit den verdampften Elementen beschichtet. Das Aufbringen mittels Abscheidung aus der Gasphase hat den Vorteil, dass auch geometrisch kompliziert geformte Bauteile wie koronare Stents auch an den Schnittkanten und in Hinterschneidungen gleichmäßig beschichtet werden können.

In einem bevorzugten Ausführungsbeispiel werden in den mit der Sr/Ca-Beschichtung beschichteten Implantatkörper Stickstoffionen implantiert. Durch diese nachträgliche Implantation von Stickstoffionen in die Sr/Ca-Beschichtung entsteht, wie oben beschrieben, in Abhängigkeit von den jeweils vorliegenden Implantationsbedingungen ein Gradient von Strontium- und/oder Calcium-Ionen über die oberflächennahen Bereiche des Implantatkörpers und der Sr/Ca-Beschichtung, der unter anderem zu einer Erhöhung der Haftfestigkeit der Sr/Ca-Beschichtung an dem Implantatkörper führt. Durch die Strahlführung bei der Ionenimplantation können auch lokale Änderungen des Konzentrationsgradienten von Strontium und/oder Calcium erreicht werden. Dies ist beispielsweise vorteilhaft in engen Radien zwischen Stegen eines Stents oder in Bohrungen von Knochenplatten. Eine lokal unterschiedliche Implantationsrate führt auch zu Unterschieden in der Eindringtiefe der Strontium- und/oder Calcium-Ionen in dem Implantatkörper. Entsprechend kann die Strahlführung bei der Ionenimplantation so gesteuert werden, dass Zonen der Implantate, die im späteren Einsatz nur eine geringe Stützfunktion aufweisen, von der Ionenimplantation ausgespart bleiben. Werden derart behandelte Implantate unter physiologischen Bedingungen eingesetzt, degradieren diese erfindungsgemäß und gewollt lokal verschieden. Somit kann die Degradationsgeschwindigkeit auch lokal gezielt eingestellt werden. Hierbei ist die Degradationsgeschwindigkeit dort am höchsten, wo die größte Konzentration an Stickstoffionen implantiert wurde, das heißt dort wo die Eindringtiefe der Strontium- und/oder Calcium-Ionen am größten ist.

In einem weiteren bevorzugten Ausführungsbeispiel wird der beschichtete Implantatkörper unter Schutzgas verpackt, um eine sofortige Oxidation der Oberfläche des beschichteten Implantats zu vermeiden.

In einem weiteren Ausführungsbeispiel kann auf die Sr/Ca-Beschichtung anschließend an die Aufbringung dieser Beschichtung oder ggf. im Anschluss an die Stickstoffionenimplantation eine zweite Beschichtung enthaltend ein sauer abbaubares Polymer aufgetragen werden, beispielsweise ein Polylactid, ein Polyglycosid oder ein Copolymer aus diesen, besonders bevorzugt PLLA oder PLGA, oder ein Blend aus den genannten Polymeren. Dieses Polymer kann zusätzlich eine pharmazeutisch aktive Substanz enthalten. Eine solche zweite Beschichtung weist die oben angegebenen Vorteile auf. Eine derartige zweite Beschichtung wird vorzugsweise mittels Aufsprühen oder mittels Tauchen in eine verdünnte Polymerlösung hergestellt. Bei der zuletzt genannten Möglichkeit wird das Lösungsmittel anschließend thermisch ausgetrieben.

Die obige Aufgabenstellung wird ferner gelöst durch ein Implantat erhältlich durch ein oben beschriebenes erfindungsgemäßes Verfahren. Ein derartiges Implantat weist die oben im Zusammenhang mit dem erfindungsgemäßen Herstellungsverfahren angegebenen Vorteile auf.

Das erfindungsgemäße Verfahren bzw. das erfindungsgemäße Implantat wird nachfolgend in Beispielen erläutert.

### 1. Beispiel:

Fertig prozessierte Stentkörper aus Reineisen 99,5 werden im entfetteten Zustand in eine Vakuumkammer verbracht. Am Boden dieser Kammer befindet sich ein Target aus einem Erdalkalimetall, z.B. Strontium. Nach Anlegen eines Vakuums von ca. 1 x 10⁻⁴ Pa wird das Sr-Target über den Schmelzpunkt von 777°C erhitzt. Das dann entstehende gasförmige Strontium schlägt sich nun allseitig in fester Form auf den Implantatkörper nieder. Eine thermische Belastung des Substrates tritt dabei nicht auf. Nach einer Bedampfungszeit von ca. 2 Stunden ist eine Schichtdicke von ca. 2 µm erreicht. Die Behandlungskammer wird anschließend mit Argon belüftet und die beschichteten Stentkörper mit Hilfe einer Schleuse unter Argon verpackt. Diese Maßnahme dient zur Verhinderung einer vorzeitigen Oxidbildung der hoch sauerstoffaffinen Strontium-Schicht.

In einer weiteren, mit Argon gefluteten Beschichtungsanlage wird der mit Strontium beschichtete Stentkörper auf einem Probenhalter so montiert, dass sich der Ionenstrahl über die gesamte äußere Oberfläche bewegen kann. Danach erfolgt eine Implantation von Stickstoffionen bei einer Energie von 40 keV und einer Dosis von ca. 4 x 10¹⁷ cm⁻². Eine Legierungsbildung zwischen Fe und Sr findet nicht statt. Allerdings wird ein Teil des implantierten Stickstoffs in der Fe-Matrix des Stentkörpers gelöst und ein weiterer Teil bildet mit dem im Stentkörper vorhandenen Eisen Fe-Nitride, welche eine Oberflächenhärtung bewirken.

Durch die bei komplizierten Stentgeometrien nicht vermeidbaren Abschattungseffekte erhält z.B. die Innenseite des bestrahlten Stentkörpers nur eine Dosis von ca. 1 x 10¹⁷ cm⁻².

Der durch die Stickstoffimplantation thermodynamisch initiierte Zwang zur Diffusion von Strontium in das Innere des Implantatkörpers, führt zu einer Erhöhung der Dicke der Srenthaltenden Deckschicht von ca. 2 µm auf ca. 3 µm. Durch die Bestrahlung mittels Stickstoffionen wird innere Reibung an der Oberfläche des Stents erzeugt, die eine mikroskopische Aufheizung der Oberfläche bewirkt. Diese begünstigt die Diffusion der Sr-Ionen. In einer Tiefe größer 3 µm ist mittels ortsaufgelöster EDX kein Sr mehr nachweisbar.

Nach der Entnahme aus der Behandlungskammer werden die Stents körperphysiologischen Bedingungen (wässriger Elektrolyt) ausgesetzt. Die nur wenig mit Stickstoffionen verfestigte Sr-Schicht der Stentinnenseite degradiert dabei langsamer als die mit der höheren Dosis bestrahlte Stentaußenoberfläche.

Der Chloridionen enthaltende wässrige Elektrolyt überführt zwar schnell das an der Innenoberfläche angeordnete Strontium der Beschichtung in das gut wasserlösliche Strontiumhydroxid. Danach sinkt jedoch der Korrosionsangriff, da eine gewisse Mikropassivierung der Eisenoberfläche durch das entstehende basische Milieu eintritt.

Anders dagegen verhält sich die Stentaußenoberfläche. Hier kann das durch Stickstoff in der Eisenmatrix des Implantatkörpers verdichtete Strontium seine Wirkung als galvanisches Element effektiv entfalten. Es kommt verstärkt zum Lochfraß und Spaltkorrosion. Diese Mechanismen treten auch noch dann verstärkt in Erscheinung, wenn die Korrosion bereits tiefer liegende Strontium-freie Zonen erreicht hat. Durch den hohen Zerklüftungsgrad wirkt dann zunehmend der Mechanismus der Flächenkorrosion. An der Stentaußenoberfläche überwiegt bei weitem die Metallauflösung gegenüber dem Repassivierungsffekt. Im Vergleich zu einem unbeschichteten Eisenstent einer identischen Zusammensetzung degradiert der so behandelte Stent in SBF (simulated body fluid) bei 37°C 1,5 mal schneller.

### 2. Beispiel:

Das 2. Beispiel wird analog zum 1. Beispiel, ebenfalls mit einem Stentkörper aus Reineisen 99,5 durchgeführt. In diesem Fall dient als Target Calcium mit einem Reinheitsgrad von 95%. Nach Anlegen eines Vakuums von ca. 1 x 10-4 Pa wird das Ca-Target über den Schmelzpunkt von 839°C erhitzt. Die weitere Vorgehensweise und die übrigen Parameter entsprechen dem 1. Beispiel.

### 3. Beispiel:

Das 3. Beispiel wird analog zum 1. Beispiel durchgeführt. Allerdings wird in diesem Beispiel ein Target bestehend aus einer Sr-Legierung mit ca. 18,25% A1 verwendet. Diese eutektische Legierung weist einen Schmelzpunkt von 590°C auf. Demzufolge kann die Verdampfungstemperatur erniedrigt werden. Alternativ kann, bei Einstellung einer höheren Temperatur, die Abscheiderate erhöht werden, so dass sich die Behandlungszeit verkürzt. Die übrigen Parameter und die Vorgehensweise entsprechen dem 1. Beispiel.

### 4. Beispiel:

Ausgehend von dem 1. oder 2. Beispiel wird auf den Prozess der Implantation von Stickstoff-Ionen verzichtet. Es werden also nicht zusätzlich Stickstoffionen implantiert. Folglich können auch nicht lokal unterschiedliche Korrosionsraten eingestellt werden. Das Implantat wirkt dann überwiegend als Sr- oder Ca-Reservoir mit den beschriebenen Vorteilen bei der Osteosynthese. Die Degradationsrate beträgt das 1,0-fache bis 1,5-fache bei der Anwendung als orthopädisches Implantat. Das konkrete Degradationsverhalten ist abhängig von der Implantatgeometrie. Filigrane Implantate wie Knochenschrauben oder Radiusplatten für Kinder degradieren auf Grund des größeren Oberflächen-/Volumenverhältnisses schneller (Faktor 1,5) als z.B. Knochenplatten für die Humerusfraktur von Erwachsenen. (Faktor 1,2).

### 5. Beispiel:

Das 5. Beispiel wird analog zu einem der Beispiele 1 bis 4 durchgeführt und nach der Aufbringung der Sr/Ca-Beschichtung oder ggf. nach der Stickstoff-Implantation, eine sauer abbaubare Polymerschicht, z.B. PLLA L 210 oder PLGA, auf der Sr/Ca-Beschichtung als sogenanntes Topcoat angeordnet. Optional kann in die Polymerschicht eine pharmazeutisch aktive Substanz eingelagert werden. Das Topcoat wird bei Normaldruck und Raumtemperatur mittels Sprühverfahren in einer Behandlungskammer aufgebracht. Danach erfolgt ein Temperprozess bei PLGA über 13h bei 40°C; bei PLLA L 210 über 13 h bei 80°C.

Der Vorteil des Topcoats besteht darin, dass bei Beginn der Degradation in vivo zunächst ein saures Abbauprodukt entsteht. Dieses tritt in Wechselwirkung mit den basisch reagierenden Sr und Ca-Schichten. In der Summe tritt eine rasche Neutralisierung der Implantatumgebung ein. Somit wird eine inflammatorische Reaktion der umgebenden Gewebezellen unterdrückt, da die Konzentration der Wasserstoffionen in der Umgebung nicht wesentlich von pH 7 abweicht.

## Patentansprüche

1. Implantat, insbesondere intraluminale Endoprothese, mit einem Implantatkörper enthaltend biodegradierbares metallisches Material, vorzugsweise Eisen, wobei mindestens ein Teil der Oberfläche des Implantatkörpers eine erste Beschichtung aufweist, welche aus einer Zusammensetzung enthaltend mindestens Calcium gebildet ist.

2. Implantat nach Anspruch 1, **dadurch gekennzeichnet, dass** die erste Beschichtung aus einer Zusammensetzung enthaltend mindestens Strontium und Calcium gebildet ist.

3. Implantat nach Anspruch 1 oder 2, **dadurch gekennzeichnet, dass** die erste Beschichtung eine Dicke von etwa 0,5 µm bis etwa 10 µm, vorzugsweise etwa 1 µm bis etwa 5 µm aufweist.

4. Implantat nach einem der vorhergehenden Ansprüche, **dadurch gekennzeichnet, dass** das Implantat in der ersten Beschichtung und ggf. auch in dem Implantatkörper implantierten Stickstoff aufweist, wobei der integrale Stickstoffanteil von der Oberfläche der Sr/Ca-Beschichtung bis in eine Tiefe von 5 µm vorzugsweise zwischen etwa 0,1 Gew.% und etwa 1,5 Gew.% beträgt.

5. Implantat nach einem der vorhergehenden Ansprüche, **dadurch gekennzeichnet, dass** der Implantatkörper vorzugsweise überwiegend Eisen, insbesondere mehr als 80 Gew.% Eisen, besonders bevorzugt mindestens 99 Gew.% Eisen enthält.

6. Implantat nach einem der vorhergehenden Ansprüche, **dadurch gekennzeichnet, dass** auf der ersten Beschichtung zusätzlich eine zweite Beschichtung enthaltend ein sauer abbaubares Polymer vorgesehen ist, welche vorzugsweise eine pharmazeutisch aktive Substanz enthält.

7. Verfahren zur Herstellung eines Implantats, insbesondere einer intraluminalen Endoprothese, mit einem Implantatkörper enthaltend biodegradierbares metallisches Material, vorzugsweise Eisen, umfassend die folgenden Schritte:
a) Bereitstellen des Implantatkörpers,
b) Beschichtung des Implantatkörpers mit einer Zusammensetzung enthaltend mindestens ein Element der Gruppe bestehend aus Strontium und Calcium, wobei die Beschichtung in Schritt b) durch Abscheidung aus der Gasphase erfolgt.

8. Verfahren nach Anspruch 7, **dadurch gekennzeichnet, dass** in den beschichteten Implantatkörper Stickstoffionen implantiert werden.

9. Verfahren nach einem der Ansprüche 7 bis 8, **dadurch gekennzeichnet, dass** der beschichtete Implantatkörper unter Schutzgas verpackt wird.

10. Verfahren nach einem der Ansprüche 7 bis 9, **dadurch gekennzeichnet, dass** auf den beschichteten Implantatkörper zusätzlich eine zweite Beschichtung enthaltend ein sauer abbaubares Polymer aufgebracht wird, welche vorzugsweise eine pharmazeutisch aktive Substanz enthält.

11. Implantat, insbesondere intraluminale Endoprothese, mit einem Körper enthaltend metallisches Material, vorzugsweise Eisen, erhältlich durch ein Verfahren gemäß einem der Ansprüche 7 bis 10.

12. Implantat, insbesondere intraluminale Endoprothese, mit einem Implantatkörper enthaltend biodegradierbares metallisches Material, wobei mindestens ein Teil der Oberfläche des Implantatkörpers eine erste Beschichtung aufweist, welche aus einer Zusammensetzung enthaltend mindestens ein Element der Gruppe bestehend aus Strontium und Calcium gebildet ist, und der Implantatkörper überwiegend Eisen und Mangan, Zink und/oder Wolfram enthält.

13. Implantat, insbesondere intraluminale Endoprothese, mit einem Implantatkörper enthaltend biodegradierbares metallisches Material, wobei mindestens ein Teil der Oberfläche des Implantatkörpers eine erste Beschichtung aufweist, welche aus einer Zusammensetzung enthaltend mindestens ein Element der Gruppe bestehend aus Strontium und Calcium gebildet ist, und wobei der Implantatkörper überwiegend Mangan, Zink oder Wolfram enthält.

## Claims

1. An implant, in particular an intraluminal endoprosthesis, comprising an implant body containing biodegradable metallic material, preferably iron, wherein at least a portion of the surface of the implant body has a first coating, which is formed from a composition containing at least calcium.

2. The implant according to claim 1, **characterized in that** the first coating is formed from a composition containing at least strontium and calcium.

3. The implant according to claim 1 or 2, **characterized in that** the first coating has a thickness of approximately 0.5 µm to approximately 10 µm, preferably approximately 1 µm to approximately 5 µm.

4. The implant according to any one of the preceding claims, **characterized in that** the implant has nitrogen implanted in the first coating and, optionally, also in the implant body, wherein the integral nitrogen portion of the surface of the Sr/Ca coating is preferably between approximately 0.1% by weight and approximately 1.5% by weight down to a depth of 5 µm.

5. The implant according to any one of the preceding claims, **characterized in that** the implant body preferably contains primarily iron, in particular more than 80% by weight, particularly preferably at least 99% by weight of iron.

6. The implant according to any one of the preceding claims, **characterized in that** a second coating is additionally provided on the first coating, said second coating containing a polymer which is degradable in an acidic environment and preferably contains a pharmaceutically active substance.

7. A method for producing an implant, in particular an intraluminal endoprosthesis, comprising an implant body containing biodegradable metallic material, preferably iron, having the following steps:
a) providing the implant body,
b) coating the implant body with a composition containing at least one element of the group composed of strontium and calcium, wherein the coating takes place in step b) via separation out of the gas phase.

8. The method according to claim 7, **characterized in that** nitrogen ions are implanted in the coated implant body.

9. The method according to any one of claims 7 to 8, **characterized in that** the coated implant body is packaged in an inert-gas environment.

10. The method according to any one of claims 7 to 9, **characterized in that** a second coating is additionally applied onto the coated implant body, said second coating containing a polymer which is degradable in an acidic environment and preferably contains a pharmaceutically active substance.

11. An implant, in particular an intraluminal endoprosthesis, comprising a body containing metallic material, preferably iron, which can be obtained by means of a method according to any one of claims 7 to 10.

12. An implant, in particular an intraluminal endoprosthesis, comprising an implant body containing biodegradable metallic material, wherein at least a portion of the surface of the implant body has a first coating, which is formed from a composition containing at least one element of the group composed of strontium and calcium, and the implant body contains primarily iron and manganese, zinc, and/or tungsten.

13. An implant, in particular an intraluminal endoprosthesis, comprising an implant body containing biodegradable metallic material, wherein at least a portion of the surface of the implant body has a first coating, which is formed from a composition containing at least one element of the group composed of strontium and calcium, and wherein the implant body contains primarily manganese, zinc or tungsten.

## Revendications

1. Implant, notamment endoprothèse intraluminale, avec un corps d'implant contenant un matériau métallique biodégradable, de préférence, du fer, où au moins une partie de la surface du corps d'implant présente un premier revêtement, lequel est formé par une composition contenant au moins du calcium.

2. Implant selon la revendication 1, **caractérisé en ce que** le premier revêtement est formé d'une composition contenant au moins du strontium et du calcium.

3. Implant selon les revendications 1 ou 2, **caractérisé en ce que** le premier revêtement présente une épaisseur entre environ 0,5 µm et environ 10 µm, de préférence, entre environ 1 µm et environ 5 µm.

4. Implant selon l'une des revendications précédentes, **caractérisé en ce que** l'implant présente de l'azote implanté dans le premier revêtement, et, éventuellement, dans le corps d'implant, où la proportion d'azote intégré entre la surface du revêtement à base de Sr/Ca jusqu'à une profondeur de 5 µm se situe de préférence entre environ 0,1 % en poids et environ 1,5 % en poids.

5. Implant selon l'une des revendications précédentes, **caractérisé en ce que** le corps d'implant contient de préférence du fer, notamment, plus de 80 % en poids de fer, de manière particulièrement préférée, au moins 99 % en poids de fer.

6. Implant selon l'une des revendications précédentes, **caractérisé en ce qu'**un deuxième revêtement contenant un polymère résorbable par voie acide, lequel contient de préférence une substance pharmaceutiquement active est en outre prévu complémentairement sur le premier revêtement.

7. Procédé de fabrication d'un implant, notamment d'une endoprothèse intraluminale, avec un corps d'implant contenant un matériau métallique biodégradable, de préférence, du fer, comprenant les étapes suivantes :
a) mise au point du corps d'implant,
b) revêtement du corps d'implant avec une composition contenant au moins un élément du groupe constitué par le strontium et le calcium, où le revêtement dans l'étape b) est effectué par un dépôt en phase vapeur.

8. Procédé selon la revendication 7, **caractérisé en ce que** des ions à base d'azote sont implantés dans le corps d'implant.

9. Procédé selon l'une des revendications 7 à 8, **caractérisé en ce que** le corps d'implant recouvert d'un revêtement est emballé sous atmosphère d'un gaz de protection.

10. Procédé selon l'une des revendications 7 à 9, **caractérisé en ce qu'**un deuxième revêtement contenant un polymère résorbable par voie acide est rapporté sur le corps d'implant recouvert du revêtement, lequel contient de préférence une substance pharmaceutiquement active.

11. Implant, notamment, endoprothèse intraluminale, avec un corps d'implant contenant un matériau métallique, de préférence, du fer, pouvant être obtenu par un procédé selon l'une des revendications 7 à 10.

12. Implant, notamment, endoprothèse intraluminale, avec un corps d'implant contenant un matériau métallique biodégradable, où au moins une partie de la surface du corps d'implant présente un premier revêtement, lequel est formé par une composition contenant au moins un élément du groupe constitué par le strontium et le calcium, et le corps d'implant contient majoritairement du fer et du manganèse, du zinc et/ou du tungstène.

13. Implant, notamment, endoprothèse intraluminale, avec un corps d'implant contenant un matériau métallique biodégradable, où au moins une partie de la surface du corps d'implant présente un premier revêtement, lequel est formé par une composition contenant au moins un élément du groupe constitué par le strontium et le calcium, et où le corps d'implant contient majoritairement du manganèse, du zinc ou du tungstème.
